# EUROPEAN PATENT APPLICATION

(11) **EP 1 297 803 A2**
(43) Date of publication of application: **02.04.2003**
(21) Application number: 02256743.2
(22) Date of filing: 27.09.2002
(51) Int. Cl.: A61F 13/02, A61F 13/00

(54) **Sealing device for wound dressings**

(30) Priority: 28.09.2001 JP 2001303823
(71) Applicant: JOHNSON & JOHNSON KABUSHIKI KAISHA, Koto-ku, Tokyo 135-0016 (JP)
(72) Inventor: Sawai, Seiichi, Sukagawa-shi, Fukushima (JP)
(74) Representative: Mercer, Christopher Paul

(57) **Abstract**

A sealing device for heat-mold pressing a laminate sheet of a thermoplastic material comprising: a rotatable mold frame roll having mold frames for sealing on roll outer surface; a thermal conductive heat-resistant belt moving synchronously at a same speed as the circumferential speed of the mold frame roll so as to hold and transfer the laminate sheet of the thermoplastic material between the belt and the mold frame roll; and a heater for heating and mold-pressing the laminate sheet of the thermoplastic material, the heater which contacts the heat-resistant belt from the opposite side of the surface contacting the laminate sheet of the thermoplastic material and presses the heat-resistant belt against the mold frame roll.

Effect

As the heat and pressure for sealing may be provided during one rotation of the mold frame roll, sealing is completed during approximate one round movement of the tape to be sealed around the mold frame roll, and the device may easily conform to high-speed rotation, may be miniaturized and further unprecedented high speed sealing is realized.

And in operation, sealing is performed by only rotational movement at a constant speed, using only a small number of consumable parts, capable of energy-saving manufacturing, easy maintenance and checking, easy, precise and accurate positioning of sealing part.

## Description

### Detailed description of the Invention

### Technical Field of the Invention

The present invention relates to a sealing device and in particular to a compact sealing device capable of processing a large amount of products which does not need a wide area or large volume for setting. The sealing device of the present invention is in particular preferred for heat-sealing of peripheries or inner surfaces of cosmetic sheets or ointments, peripheries of dressings or bandages and pad peripheries or pad central areas.

### Prior Art and Problems The Present Invention Seeks to Solve

A variety of sealing devices are used in the field of medical sheets and cosmetic sheets for a number of purposes such as melt-seal, mold-press and tight-seal. For example, Japanese Laid-Open Patent 33673/96 discloses an adhesive bandage comprising a film composing a bandage base formed by laminating a water-vapor permeable film, nonwoven fabric, adhesive layer and the like of which whole periphery is sealed by means of such as heat-sealing and press-sealing so as to prevent invasion of environmental water and the like. Japanese Laid-Open Patent No. 2000-144067 discloses an apparatus for manufacturing adhesive sheet, in the field of cosmetic sheet comprising an ointment including moisturizer and the like between a sheet and a release paper, the apparatus manufacturing adhesive sheets which prevent leak of the ointment by press-sealing the peripheries before cutting out the adhesive sheets. Also conventionally natural fibers such as water absorbable cotton and woven fabrics or nonwoven fabrics of thermoplastic fibers have been used for pad materials of wound dressings and adhesive bandages. In the case of pads composed of thermoplastic fiber or partially including thermoplastic fiber, sealing of the periphery is preferred because body fluid leak from pad to environment or invasion of environmental water into pad is prevented without deteriorating feeling of the fiber material and characteristics such as water absorbability in the central area of the pad. Japanese Laid-Open Patent No. 2001-245917 discloses such pad.

Conventionally pads for sealing for mass-production are made by simply cutting a long tape material with a linear cutter and sealed by heating and/or pressing before or after such cutting. Although such sealing devices only can produce pads in the shape of square or rectangle, pads with various shapes other than tetragons are used according to wound shapes or use applications. And it is preferred to produce easily round pads in the case of pads used for bandages for protection or treatment of corn, callus, wart, footsore, swelling and the like. Conventionally, in such cases, production efficiency was low and the price of such products became high because of individual production. Therefore there has been a problem that, in addition to pads having square or rectangular shapes that can be made by cutting a long sheet material with a linear cutter, pads with various shapes such as circular or oval shapes can be easily and efficiently sealed.

In conventional methods, most of devices adopt intermittent-motion mechanisms including processes such as transferring a material to be sealed to a sealing device, stopping the transferring motion, positioning the material, and then sealing it with a fixed pressing machine. In such systems including transferring, positioning and pressing processes, energy losses are significant because of intermittent motions, and further positioning for seal is rather difficult and often deviates from aimed positions and much labors are necessary for checking and maintenance. Also there are problems in such systems such that long horizontal spaces are necessary because the transfer of the long materials or pads after cutting is mainly horizontal movement, and subsidiary devices such as driving machines and cutting ones must be attached and they also occupy large volumes, in particular, to raise the production amount, proportional large-size systems are necessary.

In high-speed sealing in the case of intermittent systems to improve production efficiency, drawbacks such as low sealing strength, deviation of sealing position and shape distortion often take place and it is difficult to improve the production efficiency.

### Means for Solving the Problems

The present invention relates to a sealing device for heat-mold pressing a laminate sheet of a thermoplastic material comprising: a rotatable mold frame roll having mold frames for sealing on roll outer surface; a thermal conductive heat-resistant belt synchronously moving at a same speed with the circumferential speed of the mold frame roll so as to hold and transfer the laminate sheet of the thermoplastic materials between the belt and the mold frame roll; and a heater for heating and mold-pressing the laminate sheet of the thermoplastic material, the heater which contacts the heat-resistant belt from the opposite side of the surface contacting the laminate sheet of the thermoplastic materials and presses the heat-resistant belt against the mold frame roll, and further to a sealing device for heat-mold-pressing a fibrous cotton material and a thermoplastic material comprising: a rotatable mold frame roll having mold frame for sealing on outer surface; a thermal conductive heat-resistant belt moving at a same speed synchronously with the circumferential speed of the mold frame roll so as to hold and transfer the laminated fibrous cotton material and the thermoplastic material between the belt and the mold frame roll; and a heater for heating and mold-pressing the laminated fibrous cotton material and the thermoplastic material, the heater which contacts the heat-resistant belt from the opposite side of the surface contacting the laminated fibrous cotton material and the thermoplastic material and presses the heat-resistant belt against the mold frame roll. And further the present invention relates to said sealing device wherein said heater comprises a plurality of heater blocks and respective heater blocks are temperature-controllable.

The thermoplastic materials of the present invention include widely so-called thermoplastic elastomers and thermoplastic resins. The laminate sheet of thermoplastic materials may be a laminate sheet of more than one thermoplastic elastomers, a laminate sheet of more than one thermoplastic resins and a laminate sheet of a thermoplastic elastomer and a thermoplastic resin. Further a material that has no thermo plasticity may be incorporated to the laminate sheet as far as heat seal of the laminate sheet may be attained.

The fibrous cotton material of the present invention is a material for a pad in the case of adhesive bandage for absorbing body fluids bleeding out of injured part such as wound or a material for absorbing or holding drugs and the like in the case of ointment. The materials are not in particular restricted and they may be natural fibers such as cottons, sponge materials such as natural sponges, various synthetic fibers or synthetic resins having water absorbability or water-vapor absorbability. The materials may be mixed with hydrocolloids, fibers or powders of high-water-absorbable materials and also with thermoplastic fibers to enhance heat-sealing property. As the hydrocolloids, water-soluble gums such as pectin, guar gum and xanthan gum, gelatin, carboxymethyl cellulose (CMC), sodium CMC, sodium alginate, calcium alginate, polysaccharides and the like may be exemplified. As high-water-absorbable materials, starch graft copolymers such as starch graft copolymers of acrylates, acryl amide salts and polyacrylate may be exemplified.

In the case of wound dressing, the dressing itself or a layer thereof may contain a medicament or an active ingredient to provide wounded part with water or to accelerate cure of the wound. Saline solution such as physiological saline may be adopted to provide the wounded part with water. The medicament and physiologically active ingredient may be in the form of solution, suspension, cream, ointment, gel, powder, granule and the like, and more concretely antiseptic solutions, antibiotics such as tetracycline, erythromycin and gentamicin may be exemplified.

The thermoplastic material of the present invention may be a sheet in the form of net or porous sheet to cover the fibrous cotton material to prevent uneven distribution or dishevelment of the fibrous cotton material or to prevent agglutination or adhesion of the cotton material to wound surface. Consequently in such case, the thermoplastic material may preferably be a material that allows easily to permeate blood or body fluid, that has low affinity with blood or body fluid and that does not adhere to the wound when they coagulate or get dry. Such thermoplastic material may be polyolefins such as polyethylene and polypropylene and polyesters such as polyethylene terephthalate and polybutylene terephthalate.

Sealing is performed during the process that the laminate body of thermoplastic materials is held between the heat-resistant belt and the outer surface of the mold frame roll. One long sheet of thermoplastic material and another long sheet of different thermoplastic material are laminated before or when they reach the mold frame roll, they are inserted and held between the mold frame roll and the heat-resistant belt and transferred in concert with their rotation. The heat-resistant belt is driven by a heat-resistant belt driving gear through intermediate gears from the main gear which drives the mold frame roll so that both surfaces of the mold frame roll and the heat-resistant belt move at the same velocity. The mold frame roll may be installed so as to rotate with its own driving device as said above or to rotate together with the heat-resistant belt using a rate controllable driving device such as servo-motor of the driving gear of the heat-resistant belt. Mold frames for sealing are aligned on outer surface of the mold frame roll.

The heat-resistant belt may preferably be formed of a shape-stable heat-conductive material such as a thin metal plate of stainless steel. The surface of the heat-resistant belt is flat. The heat-resistant belt is heated and pressed by a heater block from outside and the laminate sheet of thermoplastic material is pressed and sealed between the mold frame roll and the heat-resistant belt. The heater block may be a single block or may be divided into some blocks. The heater block presses the laminate sheet through the heat-resistant belt toward central axis of the mold frame roll while heating. Pressing pressure is appropriately determined in relation to sealing rate, that is, a rotation rate of the mold frame roll and the pressure may be from about 2 kg/cm² to about 5 kg/cm². In case less than 2 kg/cm², sealing may sometimes be insufficient.

The surface temperature of the heater block is appropriately set considering conditions such as melting points and thicknesses of the thermoplastic materials, thickness of the cotton material and usually in the range of 120 and 23°C. In case of a plurality of heater blocks, each heater block may have independent temperature controller and is controlled within above temperature range.

Although rotation rate of the mold frame roll is not in particular restricted, practically the rate may be from about 5 rpm to about 60 rpm. In case no more than 5 rpm, productive efficiency is too low and also excessive melting of thermoplastic materials occurs because of too long heating time. Sufficient sealing is attained in the rotation rate up to 60 rpm. In case of rotation rate exceeding 60 rpm, it may be necessary to raise heater block temperatures, or sealing may be insufficient. Productive efficiency of the present device is determined by the rotation rate and circumference length, which is proportional to radius length, of the mold frame roll. Almost whole circumference of the roll may be used for sealing in the present device. Consequently improved productive efficiency is attained with significantly small-sized system comparing with conventional systems.

The shape of the mold frames on the outer surface of the mold frame roll may be not only conventional rectangular but also a circle, oval shape and any other shapes. Thereby various shapes of pads may be made according to the shape of wound or application position to be treated. For example, in the case of a pad for protecting or treating corn, callus, wart, footsore, swelling and the like, a round pad may be easily made. The device of the present invention may be used for various applications such as quilting adhesion, dotted adhesion and covering protection materials made by sealing a plastic material and a cloth.

The present invention will be described in detail with reference to drawings.

### Brief Description of Drawings

Figure 1 A descriptive drawing showing a device of the present invention.
Figure 2 A plan view of an example of arrangement of sealing mold frame of a sealing device of the present invention.

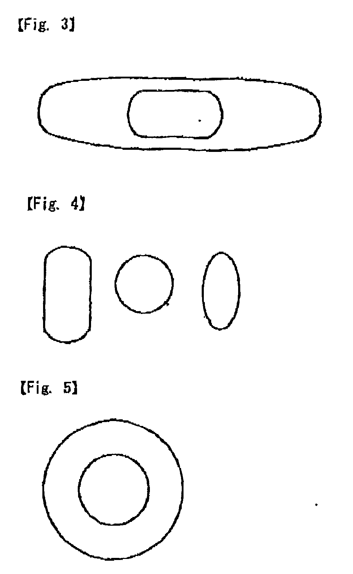

Figure 3 A plan view of an example of an adhesive bandage using a sealed pad made by a sealing device of the present invention.
Figure 4 Examples of the sealable shapes for the device of the present invention.
Figure 5 A plan view of an example of a dressing for wound using sealed pad made by sealing device of the present invention.

### Description of Symbols

1 ... a thermoplastic material, 2 ... another thermoplastic material, 3, 9 ... heat-resistant belt driving roll, 4, 10, 21, 22, 23, 24 ... heat-resistant belt auxiliary roll, 5, 11 ... heat-resistant belt, 6, 7, 8, 12, 13, 14 ... heater, 15 ... mold frame roll, 16 ... sealed pad tape

### Working Mode of the Present Invention

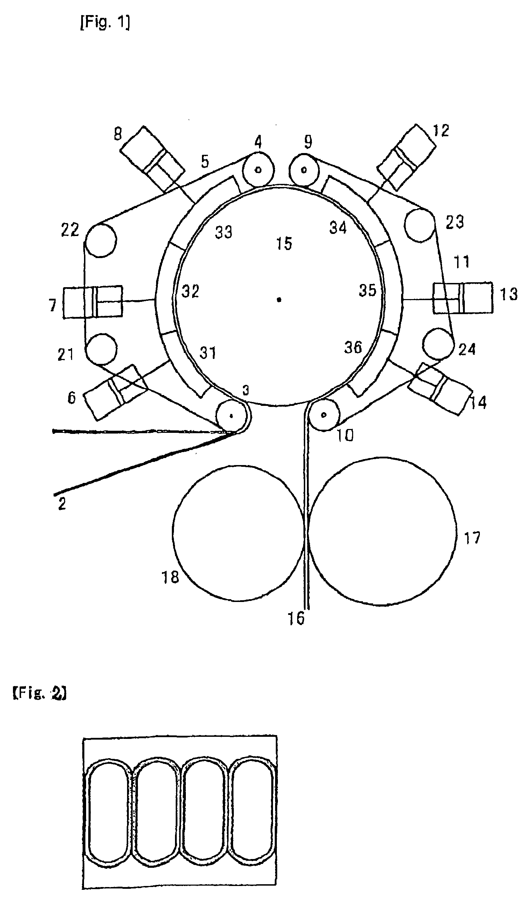

Figure 1 shows a front view of an example of a device of the present invention. In this example the heat resistant belt is divided into two parts, one an entry side belt 5 and another an exit side 11. The material of SUS 55 in thickness of 0.5 mm is used for the heat-resistant belts 5 and 11. The lengths of the belts 5 and 11 are the same and both 983 mm. They synchronously rotate at the same rate driven by respective driving roll 3 and 9. Diameters of the heat-resistant driving rolls and auxiliary rolls 21, 22, 23, 24 are all 35 mm.

The mold frame roll 15 rotates by a servo-motor synchronously with the heat-resistant belt driving rolls 3 and 6. The diameter of the mold frame roll is 245 mm. In this example, as a pad at the left side of Figure 4 is sealed, mold frames for sealing shown by oblique lines in Figure 2 are aligned according to the pad shape to be sealed on the outer surface of the mold frame roll. The pad shape is constructed such that the length of the longitudinal direction is 26 mm and the width is 12 mm, having parallel linear line segments at both sides in 20 mm lengths, and both end parts of the longitudinal direction is formed of multiple circular arcs having different radii from the linear line segment without forming any angles. The number of mold frames in this example is 64. The outer surface of the mold frame roll, that is, mold frame area is heated with a heater from inside the mold frame roll. The heating temperature is appropriately selected in the range from about 120°C to about 200°C.

The heater blocks are set along the outer surface of the mold frame roll. Three heater blocks for each heat-resistant belt are set in this example at the outer side of the heat-resistant belt. The heater blocks provide with heat for heat-seal and also pressure for pressing a laminate sheet against the mold frame. Consequently the heater block surfaces forms an arc with the same radius as the outer surface of the mold frame roll.

Three heater blocks 6, 7 and 8 for heating the entry side heat-resistant belt 5 and three heater blocks 12, 13 and 14 for heating exit side heat-resistant belt 11 have respective temperature controllers capable of independently setting optional temperatures. The temperatures of heater blocks during sealing are usually from about 120°C to about 240°C.

Although the rotation rate of the mold frame roll is not in particular restricted as far as sealing is attained, it is controlled from about 7 rpm to about 50 rpm. In this example it is preferred to control the number of heater blocks for heating and further to set temperatures properly according to the rotation rate of the mold frame roll. Controlling the number of heater blocks for heating enables to maintain the heating time for sealing constant and to uniform the sealing conditions. For example, in the case of 500 per minute production rate (rotation rate of the mold frame roll: about 8 rpm), only one heater block is heated. And one heating heater block is added per 500 per minute increase of production amount and all six heater blocks are heated in production amount of 3000 per minute (rotation rate of mold frame roll: about 47 round per minute). In this example, setting temperatures for respective heater blocks in the case of production rate of 3,000 per minute are, for example, heater block 6: 150°C, 7: 220°C, 8: 220°C, heater block 12: 220°C, 13: 220°C, 14: 220°C, and the heater inside the mold frame roll is set at 90°C in this case.

In this example, a net of polyethylene, Delnet (registered trademark), is used for thermoplastic material 1 and a nonwoven fabric made from polyester and polyethylene is used for fibrous cotton material 2. The thermoplastic material tape 1 and fibrous cotton material tape 2 are laminated on the heat-resistant belt driving roll 3 and transferred while holding between the heat resistant belt 5 and the mold frame roll 15 running at the same speed. All the heater blocks push the heat-resistant belt at the pressure of 2 kg/cm² to seal the pad material. As described above, as the pad is sealed between the heat-resistant belt and the mold frame roll running at the same speed, no useless tension is imposed on the pad material, there is no mechanical part which moves rapid reciprocal movement, the device may easily conform to mass-production of articles and be miniaturized. And in operation, processes are performed by only rotation movement at a constant rate, using only a small number of consumable parts, capable of energy-saving manufacturing of articles, easy maintenance and checking, easy, precise and accurate positioning of sealing part. Further by altering sealing mold shape, sealing of optional shape may be performed at low cost. That is, it is possible to run the mold frame roll constantly at a rotation rate of 50 rpm in this example, and in this case 3200 pads per minute may be sealed. The sealed pad tape 16 is sent to a proper pad cutting process by transfer rolls 17 and 18.

### Effect of the Invention

As described above, the sealing device of the present invention, different from conventional intermittent transfer, stop and seal type systems, is capable of smooth continuous operation, and as the heat and pressure for sealing may be provided during one rotation of the mold frame roll, sealing is completed during approximate one round movement around the mold frame roll of the tape to be sealed, and the device may easily conform to high-speed rotation, may be miniaturized and further unprecedented high speed sealing is realized. And in operation, work is done by only rotational movements at a constant rate, using only a small number of consumable parts, capable of energy-saving manufacturing, easy maintenance and checking, easy, precise and accurate positioning of sealing part.

## Claims

1. A sealing device for heat-mold pressing a laminate sheet of a thermoplastic material comprising:
a rotatable mold frame roll having mold frames for sealing on roll outer surface;
a thermal conductive heat-resistant belt synchronously moving at a same speed with the circumferential speed of the mold frame roll so as to hold and transfer the laminate sheet of the thermoplastic materials between the belt and the mold frame roll; and
a heater for heating and mold-pressing the laminate sheet of the thermoplastic material, the heater which contacts the heat-resistant belt from the opposite side of the surface contacting the laminate sheet of the thermoplastic materials and presses the heat-resistant belt against the mold frame roll.

2. A sealing device for heat-mold-pressing a fibrous cotton material and a thermoplastic material comprising:
a rotatable mold frame roll having mold frame for sealing on outer surface;
a thermal conductive heat-resistant belt synchronously moving at a same speed with the circumferential speed of the mold frame roll so as to hold and transfer the laminated fibrous cotton material and the thermoplastic material between the belt and the mold frame roll; and
a heater for heating and mold-pressing the laminated fibrous cotton material and the thermoplastic material, the heater which contacts the heat-resistant belt from the opposite side of the surface contacting the laminated fibrous cotton material and the thermoplastic material and presses the heat-resistant belt against the mold frame roll.

3. The sealing device according to claim 1 or 2 wherein said heater comprises a plurality of heater blocks and respective heater blocks are temperature-controllable.

4. The sealing device according to claim 2 or 3 wherein said laminated fibrous cotton material and the thermoplastic material compose a pad material for adhesive bandage or dressing.
